(19) Europäisches Patentamt
European Patent Office
Office européen des brevets

(11) EP 3 885 452 A1

(12) **EUROPEAN PATENT APPLICATION**

(43) Date of publication:
**29.09.2021 Bulletin 2021/39**

(51) Int Cl.:
***C12Q 1/6886*** *(2018.01)*

(21) Application number: **20164814.4**

(22) Date of filing: **23.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **HOFFMANN, Ralf Dieter**
  **5656 AE Eindhoven (NL)**
- **VAN LIESHOUT, Ron Martinus Laurentius**
  **5656 AE Eindhoven (NL)**
- **STOFFELS, Monique**
  **5656 AE Eindhoven (NL)**

(74) Representative: **Philips Intellectual Property & Standards**
**High Tech Campus 5**
**5656 AE Eindhoven (NL)**

# (54) PREDICTION OF RADIOTHERAPY RESPONSE FOR PROSTATE CANCER SUBJECT BASED ON CHEMOKINE GENES

(57) The invention relates to a method of predicting a response of a prostate cancer subject to radiotherapy, comprising determining or receiving the result of a determination of a gene expression profile for each of one or more chemokine genes selected from the group consisting of: CCL2 and CXCL17, said gene expression profile(s) being determined in a biological sample obtained from the subject, and determining the prediction of the radiotherapy response based on the gene expression profile(s) for the one or both chemokine genes.

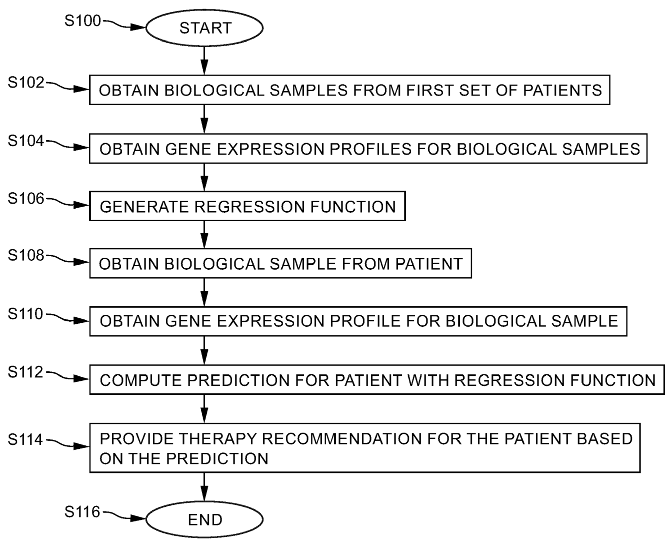


FIG. 1

EP 3 885 452 A1

## Description

FIELD OF THE INVENTION

The invention relates to a method of predicting a response of a prostate cancer subject to radiotherapy. Moreover, the invention relates to a diagnostic kit, to a use of the kit in a method of predicting a response of a prostate cancer subject to radiotherapy, to a use of a gene expression profile for each of one or more chemokine genes in radiotherapy prediction for a prostate cancer subject, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol. 68, No. 6, pages 394-424, 2018). In the US, about 90% of the new cases concern localized cancer, meaning that metastases have not yet been formed (see ACS (American Cancer Society), "Cancer Facts & Figures 2010", 2010).

For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid).

After treatment, prostate cancer antigen (PSA) levels in the blood are measured for disease monitoring. An increase of the blood PSA level provides a biochemical surrogate measure for cancer recurrence or progression. However, the variation in reported biochemical progression-free survival (bPFS) is large (see Grimm P. et al., "Comparative analysis of prostate-specific antigen free survival outcomes for patients with low, intermediate and high risk prostate cancer treatment by radical therapy. Results from the Prostate Cancer Results Study Group", BJU Int, Suppl. 1, pages 22-29, 2012). For many patients, the bPFS at 5 or even 10 years after radical RT may lie above 90%. Unfortunately, for the group of patients at medium and especially higher risk of recurrence, the bPFS can drop to around 40% at 5 years, depending on the type of RT used (see Grimm P. et al., 2012, ibid).

A large number of the patients with primary localized prostate cancer that are not treated with RT will undergo RP (see ACS, 2010, ibid). After RP, an average of 60% of patients in the highest risk group experience biochemical recurrence after 5 and 10 years (see Grimm P. et al., 2012, ibid). In case of biochemical progression after RP, one of the main challenges is the uncertainty whether this is due to recurring localized disease, one or more metastases or even an indolent disease that will not lead to clinical disease progression (see Dal Pra A. et al., "Contemporary role of postoperative radiotherapy for prostate cancer", Transl Androl Urol, Vo. 7, No. 3, pages 399-413, 2018, and Herrera F.G. and Berthold D.R., "Radiation therapy after radical prostatectomy: Implications for clinicians", Front Oncol, Vol. 6, No. 117, 2016). RT to eradicate remaining cancer cells in the prostate bed is one of the main treatment options to salvage survival after a PSA increase following RP. The effectiveness of salvage radiotherapy (SRT) results in 5-year bPFS for 18% to 90% of patients, depending on multiple factors (see Herrera F.G. and Berthold D.R., 2016, ibid, and Pisansky T.M. et al., "Salvage radiation therapy dose response for biochemical failure of prostate cancer after prostatectomy - A multi-institutional observational study", Int J Radiat Oncol Biol Phys, Vol. 96, No. 5, pages 1046-1053, 2016).

It is clear that for certain patient groups, radical or salvage RT is not effective. Their situation is even worsened by the serious side effects that RT can cause, such as bowel inflammation and dysfunction, urinary incontinence and erectile dysfunction (see Resnick M.J. et al., "Long-term functional outcomes after treatment for localized prostate cancer", N Engl J Med, Vol. 368, No. 5, pages 436-445, 2013, and Hegarty S.E. et al., "Radiation therapy after radical prostatectomy for prostate cancer: Evaluation of complications and influence of radiation timing on outcomes in a large, population-based cohort", PLoS One, Vol. 10, No. 2, 2015). In addition, the median cost of one course of RT based on Medicare reimbursement is $ 18,000, with a wide variation up to about $ 40,000 (see Paravati A.J. et al., "Variation in the cost of radiation therapy among medicare patients with cancer", J Oncol Pract, Vol. 11, No. 5, pages 403-409, 2015). These figures do not include the considerable longitudinal costs of follow-up care after radical and salvage RT.

An improved prediction of effectiveness of RT for each patient, be it in the radical or the salvage setting, would improve therapy selection and potentially survival. This can be achieved by 1) optimizing RT for those patients where RT is predicted to be effective (e.g., by dose escalation or a different starting time) and 2) guiding patients where RT is predicted not to be effective to an alternative, potentially more effective form of treatment. Further, this would reduce suffering for those patients who would be spared ineffective therapy and would reduce costs spent on ineffective therapies.

Numerous investigations have been conducted into measures for response prediction of radical RT (see Hall W.A. et al., "Biomarkers of outcome in patients with localized prostate cancer treated with radiotherapy", Semin Radiat Oncol, Vol. 27, pages 11-20, 2016, and Raymond E. et al., "An appraisal of analytical tools used in predicting clinical outcomes following radiation therapy treatment of men with prostate cancer: A systematic review", Radiat Oncol, Vol. 12, No. 1, page 56, 2017) and SRT (see Herrera F.G. and Berthold D.R., 2016, ibid). Many of these measures depend on the concentration of the blood-based biomarker PSA. Metrics investigated for prediction of response before start of RT (radical as well as salvage) include the absolute value of the PSA concentration, its absolute value relative to the prostate volume, the absolute increase over a certain time and the doubling time. Other frequently considered factors are the Gleason score and the clinical tumour stage. For the SRT setting, additional factors are relevant, e.g., surgical margin status, time to recurrence after RP, pre-/peri-surgical PSA values and clinico-pathological parameters.

Although these clinical variables provide limited improvements in patient stratification in various risk groups, there is a need for better predictive tools.

A wide range of biomarker candidates in tissue and bodily fluids has been investigated, but validation is often limited and generally demonstrates prognostic information and not a predictive (therapy-specific) value (see Hall W.A. et al., 2016, ibid). A small number of gene expression panels is currently being validated by commercial organizations. One or a few of these may show predictive value for RT in future (see Dal Pra A. et al., 2018, ibid).

In conclusion, a strong need for better prediction of response to RT remains, for primary prostate cancer as well as for the post-surgery setting.

## SUMMARY OF THE INVENTION

It is an objective of the invention to provide a method of predicting a response of a prostate cancer subject to radiotherapy, which allows to make better treatment decisions. It is a further objective of the invention to provide a diagnostic kit, a use of the kit in a method of predicting a response of a prostate cancer subject to radiotherapy, a use of a gene expression profile for each of one or more chemokine genes in radiotherapy prediction for a prostate cancer subject, and a corresponding computer program product.

In a first aspect of the present invention, a method of predicting a response of a prostate cancer subject to radiotherapy is presented, comprising:

- determining or receiving the result of a determination of a gene expression profile for each of one or both chemokine genes selected from the group consisting of: CCL2 and CXCL17, said gene expression profile(s) being determined in a biological sample obtained from the subject,
- determining the prediction of the radiotherapy response based on the gene expression profile(s) for the one or both chemokine genes, and
- optionally, providing the prediction or a therapy recommendation based on the prediction to a medical caregiver or the subject.

In recent years, the importance of the immune system in cancer inhibition as well as in cancer initiation, promotion and metastasis has become very evident (see Mantovani A. et al., "Cancer-related inflammation", Nature, Vol. 454, No. 7203, pages 436-444, 2008, and Giraldo N.A. et al., "The clinical role of the TME in solid cancer", Br J Cancer, Vol. 120, No. 1, pages 45-53, 2019). The immune cells and the molecules they secrete form a crucial part of the tumour microenvironment and most immune cells can infiltrate the tumour tissue. The immune system and the tumour affect and shape one another. Thus, anti-tumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (see Giraldo N.A. et al., 2019, ibid).

The principles described above with regard to the role of the immune system in cancer in general also apply to prostate cancer. Chronic inflammation has been linked to the formation of benign as well as malignant prostate tissue (see Hall W.A. et al., 2016, ibid) and most prostate cancer tissue samples show immune cell infiltrates. The presence of specific immune cells with a pro-tumour effect has been correlated with worse prognosis, while tumours in which natural killer cells were more activated showed better response to therapy and longer recurrence-free periods (see Shiao S.L. et al., "Regulation of prostate cancer progression by tumor microenvironment", Cancer Lett, Vol. 380, No. 1, pages 340-348, 2016).

While a therapy will be influenced by the immune components of the tumour microenvironment, RT itself extensively affects the make-up of these components (see Barker H.E. et al., "The tumor microenvironment after radiotherapy: Mechanisms of resistance or recurrence", Nat Rev Cancer, Vol. 15, No. 7, pages 409-425, 2015). Because suppressive cell types are comparably radiation-insensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is as yet uncertain, but its potential for enhancement of cancer immunotherapies is being investigated. The present invention is based on the idea that, since the status of the immune system and of the immune microenvironment have an impact on therapy effectiveness, the ability to identify markers predictive for this effect might help to be better able to predict overall RT response.

Chemokines are small, secreted proteins that are best known for their roles in mediating immune cell trafficking and lymphoid tissue development. The chemokines are the largest subfamily of cytokines and can be further subdivided into four main classes depending on the location of the first two cysteine (C) residues in their protein sequence: namely, the CC-chemokines, the CXC-chemokines, C-chemokines and CX3C-chemokines.

There is an important degree of redundancy in the chemokine superfamily, with many ligands binding different receptors (and vice versa). In the tumour microenvironment, chemokines can be expressed by tumour cells and other cells, including immune cells and stromal cells. In response to specific chemokines, different immune cell subsets migrate into the tumour microenvironment and regulate tumour immune responses in a spatiotemporal manner. In addition, chemokines can directly target non-immune cells - including tumour cells and vascular endothelial cells - in the tumour microenvironment, and they have been shown to regulate tumour cell proliferation, cancer stem-like cell properties, cancer invasiveness and meta stasis. Therefore, chemokines directly and indirectly affect tumour immunity, shape tumour immune and biological phenotypes, and influence cancer progression, therapy and patient outcomes (see Nagarsheth N. et al., "Chemokines in the cancer microenvironment and their relevance in cancer immunotherapy", Nat Rev Immunol, Vol. 17, No. 9, pages 559-572, 2017).

Chemokines can directly and indirectly target tumour stem-like cells and stromal cells in tumours. Chemokine-chemokine receptor signalling pathways affect tumour cell proliferation, sternness and angiogenesis to ultimately alter tumour metastasis and disease outcomes in patients. The expression is regulated by cancer-intrinsic genetic and epigenetic mechanism and by environmental cues in the tumour microenvironment. For example, CCL18 can directly influence tumour cells by, for example, promoting invasion, metastasis and EMT in breast cancer, pancreatic cancer, ovarian cancer and prostate cancer (see Nagarsheth N. et al., 2017, ibid). Additionally, CXCL17 is expressed in breast and colon cancer and acts as a chemoattractant for monocytes, macrophages and mature and immature DCs, thereby playing an important role in angiogenesis. However, the exact mechanism and function of CXCL17 is still not clear (see Heras S.C. las and Martinez-Balibrea E., "CXC family of chemokines as prognostic or predictive biomarkers and possible drug targets in colorectal cancer", World Journal of Gastroenterology, Vol. 24, No. 42, pages 4738-4749, 2018).

Several investigations have been performed to the effect of RT on the so-called "abscopal effect" and other immunological activation resulting from RT (see Herrera F.G. et al., "Radiotherapy combination opportunities leveraging immunity for the next oncology practice", CA: A Cancer Journal for Clinicians, Vol. 67, No. 1, 2017, pages 65-85, and Jarosz-Biej M. et al., "Tumor microenvironment as a "game changer" in cancer radiotherapy", IJMS, Vol. 20, No. 13, page 3212, 2019) indeed underlies the effect RT on modulation of the immunological pathways, enhancing the T-cell homing, engraftment and function in tumours. However, the focus is usually on the enhancement of immunotherapy (e.g., checkpoint inhibitors) and or immune response (see Connolly K.A. et al., "Increasing the efficacy of radiotherapy by modulating the CCR2/CCR5 chemokine axes", Oncotarget, Vol. 7, No. 52, pages 86522-86535, 2016).

Chemokines have been proposed as biomarkers for prostate cancer. Tsaur I. et al., "CCL2 chemokine as a potential biomarker for prostate cancer: A pilot study", Cancer Res Treat, Vol. 47, No. 2, pages 306-312, 2014, show that CCL2, CCR6, CCL5, CCL20 and CX3CL1 can be potentially used as biomarkers for neoplastic transformation. Moreover, CCR2 revealed a significant negative linear correlation with the Gleason score and grading. Chemokines have also been suggested for prognosis of RT. CXCL10 expression, for example, has been used to determine response to RT for patient with tongue tumours (see Rentoft M. et al., "Expression of CXCL10 is associated with response to radiotherapy and overall survival in squamous cell carcinoma of the tongue", Tumor Biol, Vol. 35, No. 5, pages 4191-4198, 2014).

The identified chemokine genes CCL2 and CXCL17 were identified as follows: A group of 538 prostate cancer patients were treated with RP and the prostate cancer tissue was stored. A number of these patients experienced biochemical recurrence and was treated with SRT. For 151 of these patients, the RNA expressed in the originally stored prostate cancer tissue was analysed using RNA sequencing. The mRNA expression of chemokines and their receptors was compared for the 26 out of 151 patients that died due to prostate cancer, versus the 125 out of 151 patients who survived. For the two molecules CCL2 and CXCL17, the expression level was significantly different for the survivors, suggesting that they have value in the prediction of survival after SRT.

The term "CCL2" refers to the C-C Motif Chemokine Ligand 2 gene (Ensembl: ENSG00000108691), for example, to the sequence as defined in NCBI Reference Sequence NM_002982, specifically, to the nucleotide sequence as set forth in SEQ ID NO:1, which corresponds to the sequence of the above indicated NCBI Reference Sequence of the CCL2 transcript, and also relates to the corresponding amino acid sequence for example as set forth in SEQ ID NO:2, which corresponds to the protein sequence defined in NCBI Protein Accession Reference Sequence NP_002973 encoding the CCL2 polypeptide.

The term "CCL2" also comprises nucleotide sequences showing a high degree of homology to CCL2, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 1 or amino acid sequences being at least 75%, 80%, 85%, 90%, 91%, 92%, 93%, 94%, 95%, 96%, 97%, 98% or 99% identical to the sequence as set forth in SEQ ID NO:2 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:2 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1.

The term "CXCL17" refers to the C-X-C Motif Chemokine Ligand 17 gene (Ensembl: ENSG00000189377), for example, to the sequence as defined in NCBI Reference Sequence NM_198477, specifically, to the nucleotide sequence as set forth in SEQ ID NO:3, which corresponds to the sequence of the above indicated NCBI Reference Sequence of the CXCL17 transcript, and also relates to the corresponding amino acid sequence for example as set forth in SEQ ID NO:4, which corresponds to the protein sequence defined in NCBI Protein Accession Reference Sequence NP_940879 encoding the CXCL17 polypeptide.

The term "CXCL17" also comprises nucleotide sequences showing a high degree of homology to CXCL17, e.g., nucleic acid sequences being at least 75%, 80%, 85%, 90%, 91%, 92%, 93%, 94%, 95%, 96%, 97%, 98% or 99% identical to the sequence as set forth in SEQ ID NO:3 or amino acid sequences being at least 75%, 80%, 85%, 90%, 91%, 92%, 93%, 94%, 95%, 96%, 97%, 98% or 99% identical to the sequence as set forth in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:3.

The term "biological sample" or "sample obtained from a subject" refers to any biological material obtained via suitable methods known to the person skilled in the art from a subject, e.g., a prostate cancer patient. The biological sample used may be collected in a clinically acceptable manner, e.g., in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 3.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence" and/or "metastases" and/or "castration-resistant disease" and/or "prostate cancer or disease specific death".

The term "progressive prostate cancer state" means that a sample of an individual shows parameter values indicating "biochemical recurrence" and/or "clinical recurrence" and/or "metastases" and/or "castration-resistant disease" and/or "prostate cancer or disease specific death".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumour cells as measured, for example using in vivo imaging.

The term "metastases" refers to the presence of metastatic disease in organs other than the prostate.

The term "castration-resistant disease" refers to the presence of hormone-insensitive prostate cancer; i.e., a cancer in the prostate that does not any longer respond to androgen deprivation therapy (ADT).

The term "prostate cancer specific death or disease specific death" refers to death of a patient from his prostate cancer.

It is preferred that the one or both chemokine genes comprise both chemokine genes.

It is preferred that the determining of the prediction of the radiotherapy response comprises combining the gene expression profiles for the two chemokine genes with a regression function that had been derived from a population of prostate cancer subjects.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t)=H_0(t) \cdot \exp(w_1 \cdot V_1 + w_2 \cdot V_2 + w_3 \cdot V_3 + ...)$, where $V_1$, $V_2$, $V_3$ ... are predictor variables and $H_0(t)$ is the baseline hazard while H(t) is the hazard at any time t. The hazard ratio (or the risk to reach the event) is represented by $Ln[H(t)/ H_0(t)]= w_1 \cdot V_1 + w_2 \cdot V_2 + w_3 \cdot V_3 + ...$, where the coefficients or weights wi, $w_2$, $w_3$ ... are estimated by the Cox regression analysis and can be interpreted in a similar manner as for logistic regression analysis.

In one particular realization, the prediction of the radiotherapy response is determined as follows:

$$(w_1 \cdot CCL2) + (w_2 \cdot CXCL17) \qquad (1)$$

where $w_1$ and $w_2$ are weights and CCL2 and CXCL17 are the expression levels of the chemokine genes.

In one example, $w_1$ may be about -2.0 to -1.0, such as -1.6167, and $w_2$ may be about 1.0 to 2.0, such as 1.2698.

The prediction of the radiotherapy response may also be classified or categorized into one of at least two risk groups, based on the value of the prediction of the radiotherapy response. For example, there may be two risk groups, or three risk groups, or four risk groups, or more than four predefined risk groups. Each risk group covers a respective range of (non-overlapping) values of the prediction of the radiotherapy response. For example, a risk group may indicate a probability of occurrence of a specific clinical event from 0 to <0.1 or from 0.1 to <0.25 or from 0.25 to <0.5 or from 0.5 to 1.0 or the like.

It is further preferred that the determining of the prediction of the radiotherapy response is further based on one or more clinical parameters obtained from the subject.

As mentioned above, various measures based on clinical parameters have been investigated. By further basing the prediction of the radiotherapy response on such clinical parameter(s), it can be possible to further improve the prediction.

It is preferred that the one or more clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

It is further preferred that the determining of the prediction of the radiotherapy response comprises combining the gene expression profiles for the one or both chemokine genes and the one or more clinical parameters obtained from the subject with a regression function that had been derived from a population of prostate cancer subjects.

In one particular realization, the prediction of the radiotherapy response is determined as follows:

$$(w_1 \cdot CCL2) + (w_2 \cdot CXCL17) + (w_3 \cdot pGGG) \qquad (2)$$

where $w_1$ to $w_3$ are weights, CCL2 and CXCL17 are the expression levels of the chemokine genes, and pGGG is the pathological Gleason grade group.

In one example, $w_1$ may be about -1.5 to -0.5, such as -1.1968, $w_2$ may be about 1.0 to 2.0, such as 1.3024, and $w_3$ may be about 0.5 to 1.5, such as 0.8534.

It is preferred that the biological sample is obtained from the subject before the start of the radiotherapy. The gene expression profile(s) may be determined in the form of mRNA or protein in tissue of prostate cancer. Alternatively, if the chemokines are present in a soluble form, the gene expression profile(s) may be determined in blood.

It is further preferred that the radiotherapy is radical radiotherapy or salvage radiotherapy.

It is preferred that the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy. The degree to which the prediction is negative may determine the degree to which the recommended therapy deviates from the standard form of radiotherapy.

In a further aspect of the present invention, an apparatus for predicting a response of a prostate cancer subject to radiotherapy is presented, comprising:

- an input adapted to receive data indicative of a gene expression profile for each of one or both chemokine genes selected from the group consisting of: CCL2 and CXCL17, said gene expression profile(s) being determined in a biological sample obtained from the subject,
- a processor adapted to determine the prediction of the radiotherapy response based on the gene expression profile(s) for the one or both chemokine genes, and
- optionally, a providing unit adapted to provide the prediction or a therapy recommendation based on the prediction to a medical caregiver or the subject.

  In a further aspect of the present invention, a computer program product comprising instructions which, when the program is executed by a computer, cause the computer to carry out a method comprising:
- receiving data indicative of a gene expression profile for each of one or both chemokine genes selected from the group consisting of: CCL2 and CXCL17, said gene expression profile(s) being determined in a biological sample obtained from the subject,
- determining the prediction of the radiotherapy response based on the gene expression profile(s) for the one or both chemokine genes, and
- optionally, providing the prediction or a therapy recommendation based on the prediction to a medical caregiver or the subject.

In a further aspect of the present invention, a diagnostic kit is presented, comprising:

- at least one primer and/or probe for determining the gene expression profile for each of one or both chemokine genes selected from the group consisting of: CCL2 and CXCL17, in a biological sample obtained from the subject, and
- optionally, an apparatus as defined in claim 10 or a computer program product as defined in claim 11.

In a further aspect of the present invention, a use of the kit as defined in claim 12 is presented.

It is preferred that the use as defined in claim 13 is in a method of predicting a response of a prostate cancer subject to radiotherapy.

In a further aspect of the present invention, a method is presented, comprising:

- receiving a biological sample obtained from a prostate cancer subject,
- using the kit as defined in claim 12 to determine a gene expression profile for each of one or both chemokine genes selected from the group consisting of: CCL2 and CXCL17, in the biological sample obtained from the subject.

In a further aspect of the present invention, a use of a gene expression profile for each of one or both chemokine genes selected from the group consisting of: CCL2 and CXCL17, in a method of predicting a response of a prostate cancer subject to radiotherapy is presented, comprising:

- determining the prediction of the radiotherapy response based on the gene expression profile(s) for the one or both chemokine genes, and
- optionally, providing the prediction or a therapy recommendation based on the prediction to a medical caregiver or the subject.

It shall be understood that the method of claim 1, the apparatus of claim 10, the computer program product of claim 11, the diagnostic kit of claim 12, the use of the diagnostic kit of claim 13, the method of claim 15, and the use of a gene expression profile(s) of claim 16 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a flowchart of an embodiment of a method of predicting a response of a prostate cancer subject to radiotherapy.

Fig. 2 shows a ROC curve analysis of two predictive models.

Fig. 3 shows a Kaplan-Meier curve analysis of the CAPRA-S score categories. The clinical endpoint that was tested was the time to metastases (TTM) after the start of salvage radiation therapy (SRT) due to post-surgical disease recurrence.

Fig. 4 shows a Kaplan-Meier curve analysis of the Chemokine & pGGG combination model (CXC&pGGG_model). The clinical endpoint that was tested was the time to metastases (TTM) after the start of salvage radiation therapy (SRT) due to post-surgical disease recurrence.

Fig. 5 shows a Kaplan-Meier curve analysis of the CAPRA-S score categories. The clinical endpoint that was tested was the time to prostate cancer specific death (PCa Death) after the start of salvage radiation therapy (SRT) due to post-surgical disease recurrence.

Fig. 6 shows a Kaplan-Meier curve analysis of the Chemokine & pGGG combination model (CXC&pGGG model). The clinical endpoint that was tested was the time to prostate cancer specific death (PCa Death) after the start of salvage radiation therapy (SRT) due to post-surgical disease recurrence.

DETAILED DESCRIPTION OF EMBODIMENTS

**Overview Of Radiotherapy Response Prediction**

Fig. 1 shows schematically and exemplarily a flowchart of an embodiment of a method of predicting a response of a prostate cancer subject to radiotherapy. The method begins at step S100.

At step S102, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.

At step S104, a gene expression profile for each of the two chemokine genes selected from the group consisting of: CCL2 and CXCL17, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two chemokine genes.

At step S106, a regression function for assigning a prediction of the radiotherapy response is determined based on the gene expression profiles for the two chemokine genes, CCL2 and CXCL17, obtained for at least some of the biological samples obtained for the first set of patients and respective results obtained from the monitoring. In one particular realization, the regression function is determined as specified in Eq. (1) above.

At step S108, a biological sample is obtained from a patient (subject or individual). The patient can be a new patient or one of the first set.

At step S110, a gene expression profile is obtained for each of the two chemokine genes, e.g., by performing PCR on the biological sample.

At step S112, a prediction of the radiotherapy response based on the gene expression profiles for the two chemokine genes is determined for the patient using the regression function. This will be described in more detail later in the description.

At S114, a therapy recommendation may be provided, e.g., to the patient or his or her guardian, to a doctor, or to another healthcare worker, based on the prediction. To this end, the prediction may be categorized into one of a predefined set of risk groups, based on the value of the prediction. In one particular realization, the prediction of the radiotherapy response may be negative or positive for the effectiveness of the radiotherapy. If the prediction is negative, the recommended therapy may comprise one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

The method ends at S116.

In one embodiment, the gene expression profiles at steps S104 and S110 are determined by detecting mRNA expression using two or more primers and/or probes and/or two or more sets thereof.

In one embodiment, steps S104 and S110 further comprise obtaining one or more clinical parameters from the first set of patients and the patient, respectively. The one or more clinical parameters may comprise one or more of: (i) a prostate-specific antigen (PSA) level; (ii) a pathologic Gleason score (pGS); iii) a clinical tumour stage; and iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score. The regression function for assigning the prediction of the radiotherapy response that is determined in step S106 is then further based on the one or more clinical parameters obtained from at least some of the first set of patients. In step S112, the prediction of the radiotherapy response is then further based on the one or more clinical parameters, e.g., the pathological Gleason grade group (pGGG), obtained from the patient and is determined for the patient using the regression function.

The immune system interacts in a strong manner with prostate cancer, both on a systemic level and in the tumour microenvironment. Chemokines play a central role in mediating immune cell trafficking and in lymphoid tissue development. Therefore, they play an important role in the immune response to a tumor. Chemokines and their link to immune activity in the tumor microenvironment may therefore provide information on the effectiveness of RT. However, there are around 100 different molecules that play are role. To state which members of the chemokines family may have predictive value, in this application, is extremely difficult to deduce from existing literature due to the many factors that influence the exact function of chemokines.

We investigated the extent to which the expression of chemokines and their receptors in prostate cancer tissue correlates with the recurrence of disease after radical RT or SRT.

Of an initial list of more than 100 chemokines and chemokine receptors, we have focussed on 34 chemokines and chemokine receptors (TABLE 1) that were included in a detailed analysis. Of this list, we have identified two members of the family of chemokines and chemokine receptors for which the degree of expression in prostate cancer tissue significantly correlates with mortality after SRT, in a cohort of 151 prostate cancer patients. Moreover, the molecules in addition showed a significant correlation with the occurrence of metastases after SRT in the cohort of 151 patients.

TABLE 1: Chemokines included in detailed analysis.

| Gene | Description | Nucleotide Accession.Version |
|---|---|---|
| CCL18 | C-C motif chemokine ligand 18 | NC_000017.11 |
| CCL2 | C-C motif chemokine ligand 2 | NC_000017.11 |
| CCL20 | C-C motif chemokine ligand 20 | NC_000002.12 |
| CCL22 | C-C motif chemokine ligand 22 | NC_000016.10 |
| CCL25 | C-C motif chemokine ligand 25 | NC_000019.10 |
| CCL28 | C-C motif chemokine ligand 28 | NC_000005.10 |
| CCL3 | C-C motif chemokine ligand 3 | NC_000017.11 |
| CCL5 | C-C motif chemokine ligand 5 | NC_000017.11 |
| CCR2 | C-C motif chemokine receptor 2 | NC_000003.12 |
| CCR4 | C-C motif chemokine receptor 4 | NC_000003.12 |
| CCR5 | C-C motif chemokine receptor 5 (gene/pseudogene) | NC_000003.12 |
| CCR6 | C-C motif chemokine receptor 6 | NC_000006.12 |
| CCR7 | C-C motif chemokine receptor 7 | NC 000017.11 |
| CCR9 | C-C motif chemokine receptor 9 | NC_000003.12 |
| CX3CL1 | C-X3-C motif chemokine ligand 1 | NC_000016.10 |
| CX3CR1 | C-X3-C motif chemokine receptor 1 | NC_000003.12 |
| CXCL1 | C-X-C motif chemokine ligand 1 | NC_000004.12 |
| CXCL10 | C-X-C motif chemokine ligand 10 | NC_000004.12 |

(continued)

| Gene | Description | Nucleotide Accession.Version |
|---|---|---|
| CXCL11 | C-X-C motif chemokine ligand 11 | NC_000004.12 |
| CXCL12 | C-X-C motif chemokine ligand 12 | NC_000010.11 |
| CXCL13 | C-X-C motif chemokine ligand 13 | NC_000004.12 |
| CXCL14 | C-X-C motif chemokine ligand 14 | NC_000005.10 |
| CXCL17 | C-X-C motif chemokine ligand 17 | NC_000019.10 |
| CXCL2 | C-X-C motif chemokine ligand 2 | NC_000004.12 |
| CXCL5 | C-X-C motif chemokine ligand 5 | NC_000004.12 |
| CXCL6 | C-X-C motif chemokine ligand 6 | NC_000004.12 |
| CXCL8 | C-X-C motif chemokine ligand 8 | NC_000004.12 |
| CXCL9 | C-X-C motif chemokine ligand 9 | NC_000004.12 |
| CXCR1 | C-X-C motif chemokine receptor 1 | NC_000002.12 |
| CXCR2 | C-X-C motif chemokine receptor 2 | NC_000002.12 |
| CXCR3 | C-X-C motif chemokine receptor 3 | NC_000023.11 |
| CXCR4 | C-X-C motif chemokine receptor 4 | NC_000002.12 |
| CXCR5 | C-X-C motif chemokine receptor 5 | NC_000011.10 |
| CXCR6 | C-X-C motif chemokine receptor 6 | NC_000003.12 |

Based on the significant correlation with outcome after RT, we expect that the identified molecules will provide predictive value with regard to the effectiveness of radical RT and/or SRT.

TABLE 2: Univariate Cox regression analysis in a cohort with 151 prostate cancer patients. The tested endpoints in the patient cohort were a) post-treatment metastases progression free survival, and b) post-treatment disease specific survival for men undergoing salvage radiation (SRT) after post-surgical disease recurrence. The number of events and percentage per endpoint is indicated in parenthesis. The table indicates for each tested chemokine gene the association in terms of risk (Hazard Ratio) and significance (p-value) to the tested endpoint.

| **Data Set** | **(Prostate RNAseq)** | | | |
|---|---|---|---|---|
| **# Patients** | **151** | | | |
| **Outcome** | **Post-Salvage-Radiation Outcome** | | | |
| **Endpoint (# events / # patients; % events)** | **Metastases (#65/#151; 43.0%)** | | **Prostate Cancer Mortality (#26/#151; 17.2%)** | |
| **Chemokine** | **p-value** | **HR** | **p-value** | **HR** |
| **CCL2** | 0.19 | NA | 0.04 | 1.21 |
| **CXCL17** | 0.002 | 7.9 | 0.0003 | 3.6 |

CXCL17 has been shown to be more abundant in colon cancer and breast cancer tissues. Heras S.C. las and Martinez-Balibrea E., 2018, ibid, suggested that the high expression of CXCL17 might be an indicator of poor prognosis in colon cancer. However, they do not indicate CXCL17 to prostate cancer and radiation therapy. CCL2 has been shown as a potential biomarker for prostate cancer. CCL2 (in combination with CCR6) was significantly higher in tumor tissue compared to adjacent normal tissue. CCL2 was also significantly higher in the blood samples of PCa patients, compared to controls (see Tsaur I. et al., 2014, ibid). However, the authors only link CCL2 expression to a potential diagnostic marker for PCa patients (similar to that of PSA). They also noted the lack of association with tumor stage and grading, meaning that, in their view, CCL2 may not allow evaluation of tumor dissemination. In addition they do not link CCL2 to risk stratification and/or radiation therapy.

In conclusion, the combination of CCL2 and CXCL17 shows a clear added advantage in the field of prostate cancer and/or in combination with radiation therapy.

RESULTS

## Cox Regression Analysis

We then set out to test whether the combination of these two chemokines and chemokine receptors will exhibit more prognostic value. With Cox regression we modelled the expression levels of the two chemokines to prostate cancer specific death after post-surgical salvage RT either with (CXC&pGGG_model) or without (CXCmodel) the presence of the variable pathological Gleason grade group (pGGG). We tested the two models in ROC curve analysis as well as in Kaplan-Meier survival analysis.
The Cox regression functions were derived as follows:

CXC_model:

$$(w_1 \cdot CCL2) + (w_2 \cdot CXCL17)$$

CXC&pGGG model:

$$(w_1 \cdot CCL2) + (w_2 \cdot CXCL17) + (w_3 \cdot pGGG)$$

The details for the weights $w_1$ to $w_3$ are shown in the following TABLE 3.

TABLE 3: Variables and weights for the two Cox regression models, i.e., the Chemokine model (CXC model) and the Chemokine & pGGG combination model (CXC&pGGG_model); NA - not available.

| **Variable** | **Weights** | | |
|---|---|---|---|
| **Model** | | **CXC_model** | **CXC&pGGG_model** |
| **CCL2** | $w_1$ | -1.6167 | -1.1968 |
| **CXCL17** | $W_2$ | 1.2698 | 1.3024 |
| **pGGG** | $W_3$ | NA | 0.8534 |

## ROC Curve Analysis

Next, we tested the combined Cox regression model as outlined above its power to predict 10-year prostate cancer specific death after start of salvage radiation due to post-surgical disease recurrence. The performance of the model was compared to the clinical risk score CAPRA-S (see Cooperberg M.R. et al., "The CAPRA-S score: A straightforward tool for improved prediction of outcomes after radical prostatectomy", Cancer, Vol. 117, No. 22, pages 5039-5046, 2011).
Fig. 2 shows a ROC curve analysis of two predictive models. The CXC&pGGG_model (AUC=0.89) is the Cox regression model based on two chemokine genes and the pathology Gleason grade group (pGGG) information. The CAPRA_S (AUC=0.74) is the clinical CAPRA-S score (Cancer of the Prostate Risk Assessment score).

## Kaplan-Meier Survival Analysis

For Kaplan-Meier curve analysis, the Cox regression function of the combined risk model (CXC&pGGG_model) was categorized into three sub-cohorts based on the tertiles of the risk score as calculated from the CXC&pGGG model. Moreover, the performance of the clinical CAPRA-S score (Cancer of the Prostate Risk Assessment score) was analyzed.
The patient classes represent an increasing risk to experience the tested clinical endpoints of time to development of metastases (Figs. 3 and 4) or time to prostate cancer specific death (Figs. 5 and 6) since the start of salvage RT for the created combined risk model (CXC&pGGG model) and the CAPRA-S score.
Fig. 3 shows a Kaplan-Meier curve analysis of the CAPRA-S score. The clinical endpoint that was tested was the time to metastases (TTM) after the start of salvage radiation therapy (SRT) due to post-surgical disease recurrence. Patients were stratified into three groups (1) CAPRA-S scores 0-2 (low risk); 2) CAPRA-S scores 3-5 (intermediate risk);

1) CAPRA-S scores >5 (high risk)) according to their risk to experience the clinical endpoint as predicted by the respective logistic regression model (logrank p=0.0026; HR(Intermediate Risk vs. Low Risk)=10.1; 95% CI=4.8-21.2; HR(High Risk vs. Low Risk)=14.9; 95% CI=6.5-33.4). The following supplementary lists indicate the number of patients at risk for the CAPRA-S score classes analyzed, i.e., the patients at risk at any time interval +20 months after surgery are shown: Low risk: 22, 21, 21, 20, 18, 17, 11, 0, 0; Intermediate Risk: 68, 58, 53, 50, 42, 33, 31, 14, 2, 0; High risk: 45, 32, 29, 27, 22, 16, 15, 4, 1, 0.

Fig. 4 shows a Kaplan-Meier curve analysis of the CXC&pGGG model. The clinical endpoint that was tested was the time to metastases (TTM) after the start of salvage radiation therapy (SRT) due to post-surgical disease recurrence. Patients were stratified into three cohorts (1st tertile, 2nd tertile, 3rd tertile) according to their risk to experience the clinical endpoint as predicted by the CXC&pGGG regression model (logrank p<0.0001; HR(2nd tertile vs. 1st tertile)=1.1; 95% CI=0.6-2.1; HR(3rd tertile vs. 1st tertile)=4.9; 95% CI=2.3-10.5). The following supplementary lists indicate the number of patients at risk for the CXC&pGGG_model classes analyzed, i.e., the patients at risk at any time interval +20 months after surgery are shown: 1st tertile: 50, 45, 43, 43, 37, 30, 28, 20, 3, 0; 2nd tertile: 47, 42, 42, 39, 34, 29, 27, 5, 0, 0; 3rd tertile: 38, 24, 18, 16, 13, 8, 8, 4, 0, 0.

Fig. 5 shows a Kaplan-Meier curve analysis of the CAPRA-S score. The clinical endpoint that was tested was the time to prostate cancer specific death (PCa Death) after the start of salvage radiation therapy (SRT) due to post-surgical disease recurrence. Patients were stratified into three groups (1) CAPRA-S scores 0-2 (low risk); 2) CAPRA-S scores 3-5 (intermediate risk); 1) CAPRA-S scores >5 (high risk)) according to their risk to experience the clinical endpoint as predicted by the respective logistic regression model (logrank p=0.0041; HR(Intermediate Risk vs. Low Risk)=3.7; 95% CI=1.3-10.5; HR(High Risk vs. Low Risk)=9.7; 95% CI=3.1-29.8). The following supplementary lists indicate the number of patients at risk for the CAPRA-S score classes analyzed, i.e., the patients at risk at any time interval +20 months after surgery are shown: Low risk: 23, 23, 23, 23, 20, 18, 16, 10, 1, 1, 0; Intermediate Risk: 76, 74, 66, 58, 50, 41, 36, 18, 4, 2, 0; High risk: 52, 48, 41, 33, 26, 18, 15, 7, 1, 0, 0.

Fig. 6 shows a Kaplan-Meier curve analysis of the CXC&pGGG model. The clinical endpoint that was tested was the time to prostate cancer specific death (PCa Death) after the start of salvage radiation therapy (SRT) due to post-surgical disease recurrence. Patients were stratified into three cohorts (1st tertile, 2nd tertile, 3rd tertile) according to their risk to experience the clinical endpoint as predicted by the CXC&pGGG regression model (logrank p<0.0001; HR(2nd tertile vs. 1st tertile)=7.9; 95% CI=3.2-18.7; HR(3rd tertile vs. 1st tertile)=32.1; 95% CI=11.8-87.9). The following supplementary lists indicate the number of patients at risk for the CXC&pGGG_model classes analyzed, i.e., the patients at risk at any time interval +20 months after surgery are shown: 1st tertile: 61, 51, 49, 48, 42, 36, 33, 24, 5, 2, 0; 2nd tertile: 54, 54, 48, 42, 36, 31, 24, 1, ,1, 0; 3rd tertile: 46, 40, 33, 24, 18, 10, 10, 7, 0, 0, 0.

The Kaplan-Meier curve analysis as shown in Figs. 3 to 6 demonstrates the presence of different patient risk groups. The risk group of a patient is determined by the probability to suffer from the respective clinical endpoint (metastases, prostate cancer specific death) as calculated by the risk model CAPRA-S score or CXC&pGGG_ model. Depending on the predicted risk of a patient (i.e., depending on in which risk group 1 to 3 the patient may belong) different types of interventions might be indicated. In the lowest risk category, standard of care (SOC) which is SRT potentially combined with SADT (salvage androgen deprivation therapy) delivers acceptable long-term oncological control. For the middle patient risk-group, dose escalation of the applied RT and/or combination with chemotherapy might provide improved longitudinal outcomes. This is not the case for the highest patient risk group to experience any of the relevant outcomes. In this patient risk-group escalation of intervention is indicated. Options for escalation are early combination of SRT (considering higher dose regimens), SADT, and chemotherapy. Other options are alternative therapies like immunotherapies (e.g., Sipuleucil-T) or other experimental therapies.

## Discussion

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have identified molecules of which expression shows a significant relation to mortality after radical RT and SRT and therefore are expected to improve the prediction of the effectiveness of these treatments. An improved prediction of effectiveness of RT for each patient be it in the radical or the salvage setting, will improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting a response of a prostate cancer subject to radiotherapy, comprising determining or receiving the result of a determination of a gene expression profile for each of one or both chemokine genes selected from the group consisting of: CCL2 and CXCL17, said gene expression profile(s) being determined in a biological sample obtained from the subject, and determining the prediction of the radiotherapy response based on the gene expression profile(s) for the one or both chemokine genes, and optionally, providing the prediction or a therapy recommendation based on the prediction to a medical caregiver or the subject. Since the status of the immune system and of the immune microenvironment have an impact on therapy effectiveness, the ability to identify markers predictive for this effect might help to be better able to predict overall RT response. Chemokine genes play a central role in the regulation of immune activity. The identified chemokine genes were found to exhibit a significant correlation with outcome after RT, wherefore we expect that they will provide predictive value with regard to the effectiveness of radical RT and/or SRT.

**The attached Sequence Listing, entitled 2020PF00063_Sequence Listing_ST25 is incorporated herein by reference, in its entirety.**

**Claims**

1. A method of predicting a response of a prostate cancer subject to radiotherapy, comprising:

   - determining or receiving the result of a determination of a gene expression profile for each of one or both chemokine genes selected from the group consisting of: CCL2 and CXCL17, said gene expression profile(s) being determined in a biological sample obtained from the subject,
   - determining the prediction of the radiotherapy response based on the gene expression profile(s) for the one or both chemokine genes, and
   - optionally, providing the prediction or a therapy recommendation based on the prediction to a medical caregiver or the subject.

2. The method as defined in claim 1, wherein the one or both chemokine genes comprise both chemokine genes.

3. The method as defined in claim 1 or 2, wherein the determining of the prediction of the radiotherapy response comprises combining the gene expression profiles for the two chemokine genes with a regression function that had been derived from a population of prostate cancer subjects.

4. The method as defined in claim 1 or 2, wherein the determining of the prediction of the radiotherapy response is further based on one or more clinical parameters obtained from the subject.

5. The method as defined in claim 4, wherein the clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

6. The method as defined in claim 4 or 5, wherein the determining of the prediction of the radiotherapy response comprises combining the gene expression profile(s) for the one or both chemokine genes and the one or more clinical parameters obtained from the subject with a regression function that had been derived from a population of prostate cancer subjects.

7. The method as defined in any of claims 1 to 6, wherein the biological sample is obtained from the subject before the start of the radiotherapy.

8. The method as defined in any of claims 1 to 7, wherein the radiotherapy is radical radiotherapy or salvage radiotherapy.

9. The method as defined in any of claims 1 to 8, wherein the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

10. An apparatus for predicting a response of a prostate cancer subject to radiotherapy, comprising:

- an input adapted to receive data indicative of a gene expression profile for each of one or both chemokine genes selected from the group consisting of: CCL2 and CXCL17, said gene expression profile(s) being determined in a biological sample obtained from the subject,
- a processor adapted to determine the prediction of the radiotherapy response based on the gene expression profile(s) for the one or both chemokine genes, and
- optionally, a providing unit adapted to provide the prediction or a therapy recommendation based on the prediction to a medical caregiver or the subject.

11. A computer program product comprising instructions which, when the program is executed by a computer, cause the computer to carry out a method comprising:

- receiving data indicative of a gene expression profile for each of one or both chemokine genes selected from the group consisting of: CCL2 and CXCL17, said gene expression profile(s) being determined in a biological sample obtained from the subject,
- determining the prediction of the radiotherapy response based on the gene expression profile(s) for the one or both chemokine genes, and
- optionally, providing the prediction or a therapy recommendation based on the prediction to a medical caregiver or the subject.

12. A diagnostic kit, comprising:

- at least one primer and/or probe for determining the gene expression profile for each of one or both chemokine genes selected from the group consisting of: CCL2 and CXCL17, in a biological sample obtained from the subject, and
- optionally, an apparatus as defined in claim 10 or a computer program product as defined in claim 11.

13. Use of the kit as defined in claim 12.

14. The use as defined in claim 13 in a method of predicting a response of a prostate cancer subject to radiotherapy.

15. A method, comprising:

- receiving a biological sample obtained from a prostate cancer subject,
- using the kit as defined in claim 12 to determine a gene expression profile for each of one or both chemokine genes selected from the group consisting of: CCL2 and CXCL17, in the biological sample obtained from the subject.

16. Use of a gene expression profile for each of one or both chemokine genes selected from the group consisting of: CCL2 and CXCL17, in a method of predicting a response of a prostate cancer subject to radiotherapy, comprising:

- determining the prediction of the radiotherapy response based on the gene expression profile(s) for the one or both chemokine genes, and
- optionally, providing the prediction or a therapy recommendation based on the prediction to a medical caregiver or the subject.

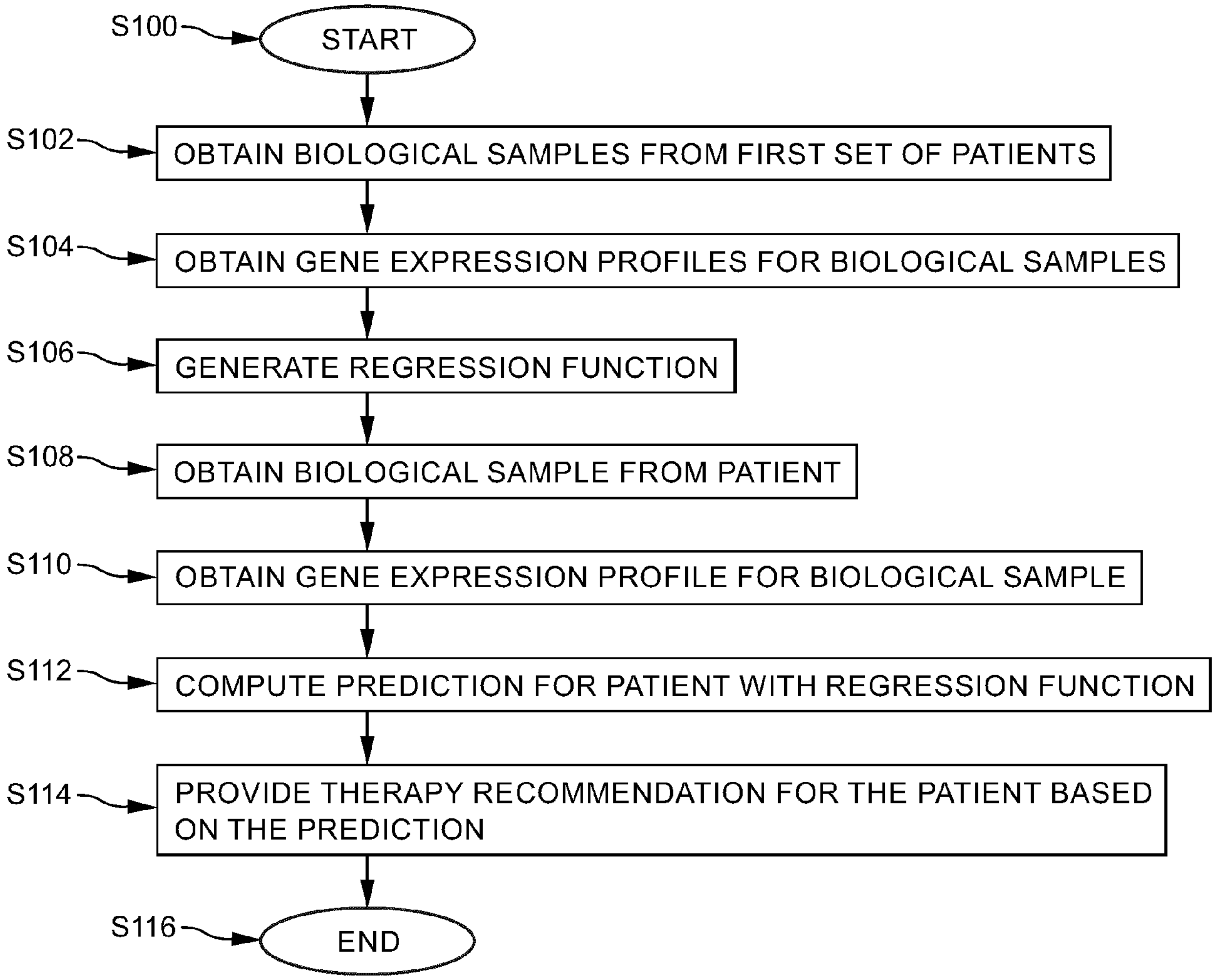
S100
START
S102
OBTAIN BIOLOGICAL SAMPLES FROM FIRST SET OF PATIENTS
S104
OBTAIN GENE EXPRESSION PROFILES FOR BIOLOGICAL SAMPLES
S106
GENERATE REGRESSION FUNCTION
S108
OBTAIN BIOLOGICAL SAMPLE FROM PATIENT
S110
OBTAIN GENE EXPRESSION PROFILE FOR BIOLOGICAL SAMPLE
S112
COMPUTE PREDICTION FOR PATIENT WITH REGRESSION FUNCTION
S114
PROVIDE THERAPY RECOMMENDATION FOR THE PATIENT BASED ON THE PREDICTION
S116
END

FIG. 1

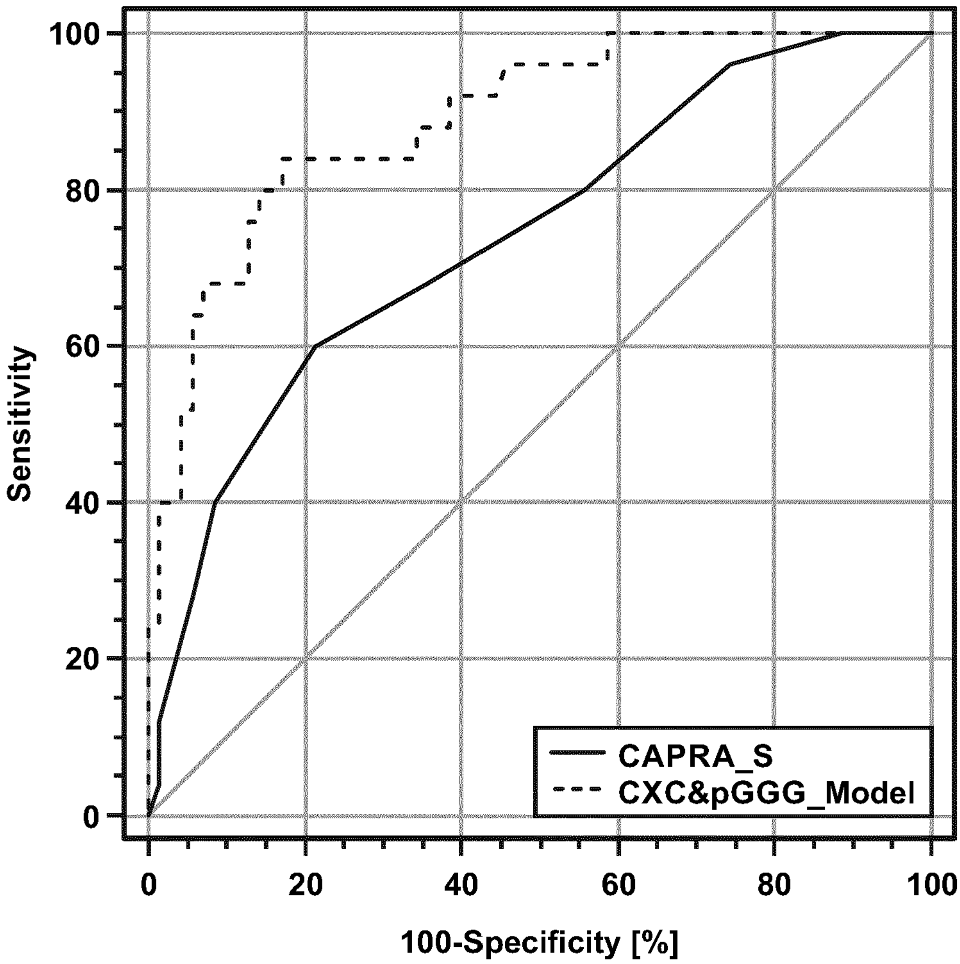
100
80
60
40
20
0
Sensitivity
0
20
40
60
80
100
100-Specificity [%]
CAPRA_S
CXC&pGGG_Model

FIG. 2

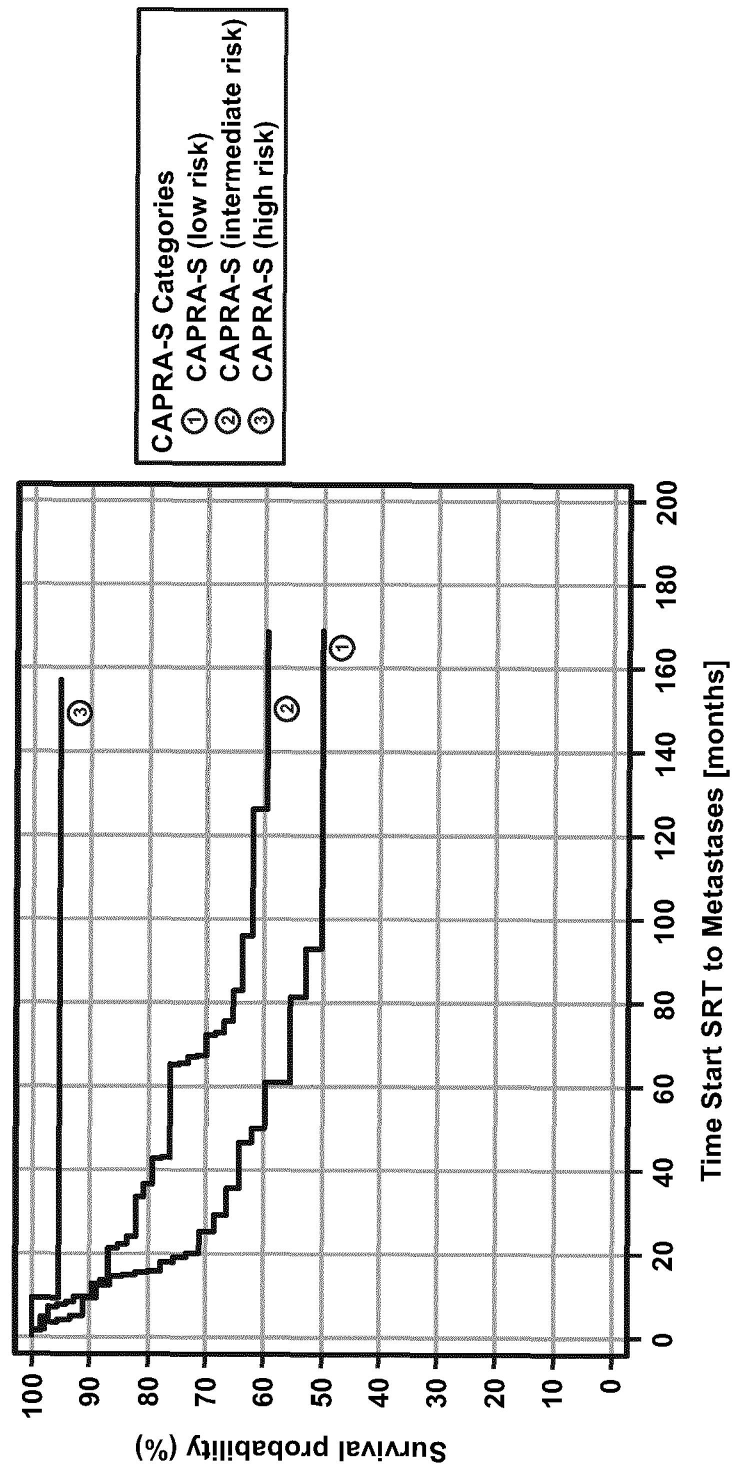
CAPRA-S Categories
① CAPRA-S (low risk)
② CAPRA-S (intermediate risk)
③ CAPRA-S (high risk)
Survival probability (%)
100
90
80
70
60
50
40
30
20
10
0
Time Start SRT to Metastases [months]
0
20
40
60
80
100
120
140
160
180
200
①
②
③

FIG. 3

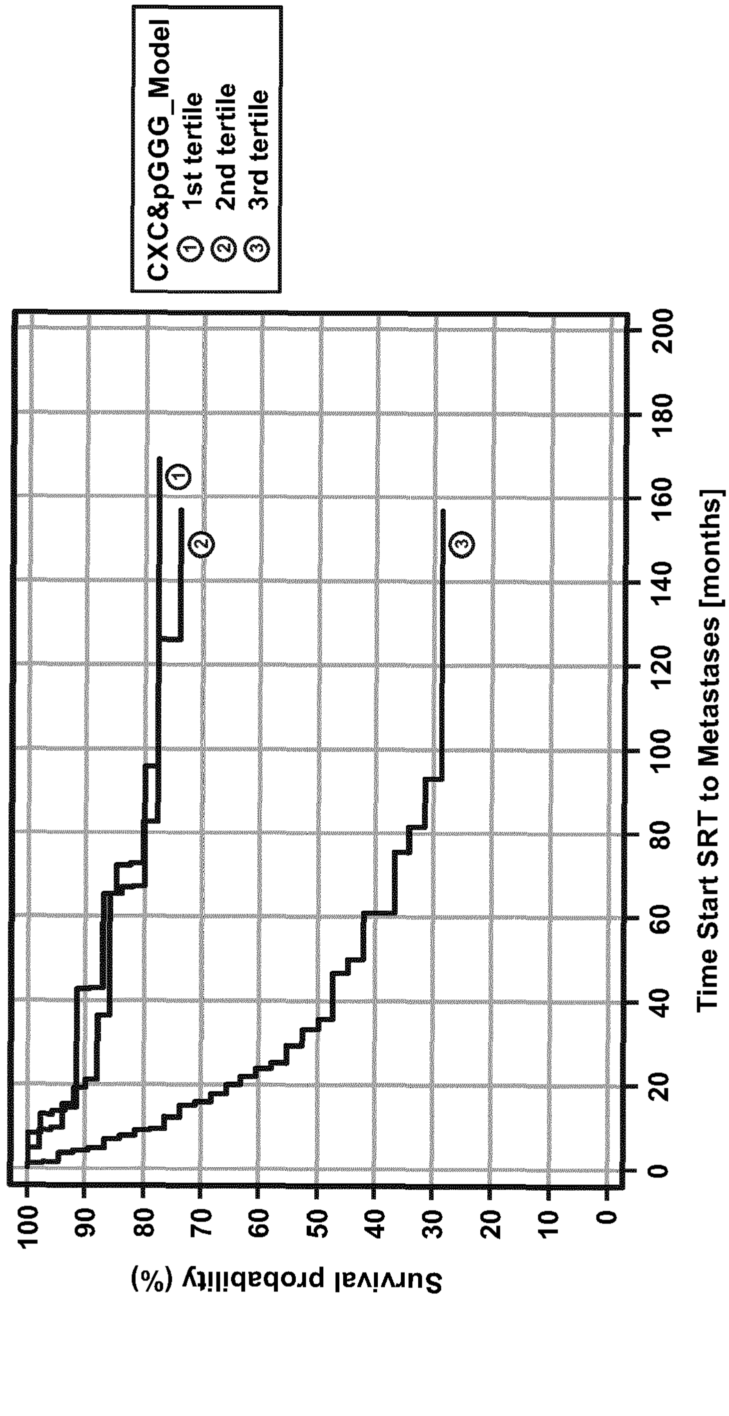
CXC&pGGG_Model
① 1st tertile
② 2nd tertile
③ 3rd tertile
Survival probability (%)
Time Start SRT to Metastases [months]
0
10
20
30
40
50
60
70
80
90
100
120
140
160
180
200
①
②
③

FIG. 4

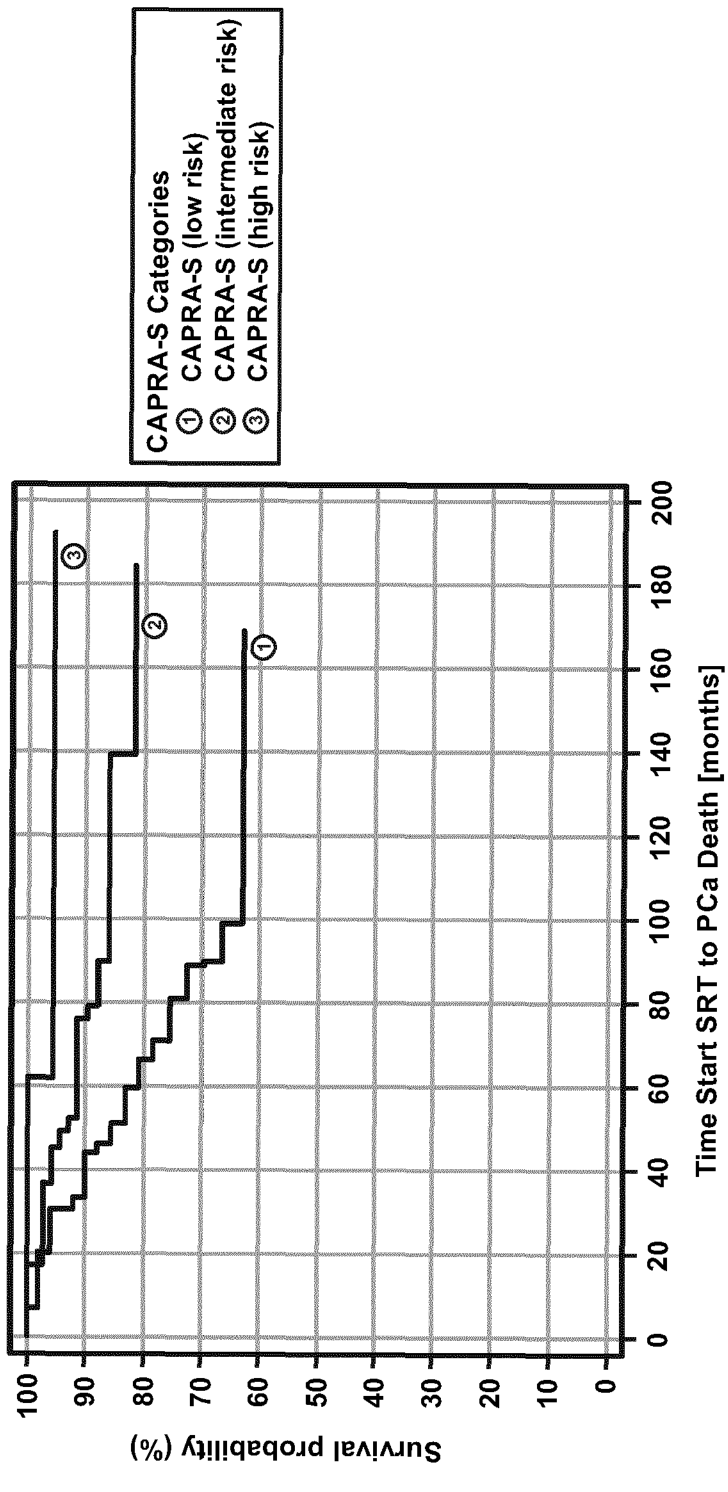
CAPRA-S Categories
① CAPRA-S (low risk)
② CAPRA-S (intermediate risk)
③ CAPRA-S (high risk)
Survival probability (%)
100
90
80
70
60
50
40
30
20
10
0
0
20
40
60
80
100
120
140
160
180
200
Time Start SRT to PCa Death [months]
①
②
③

FIG. 5

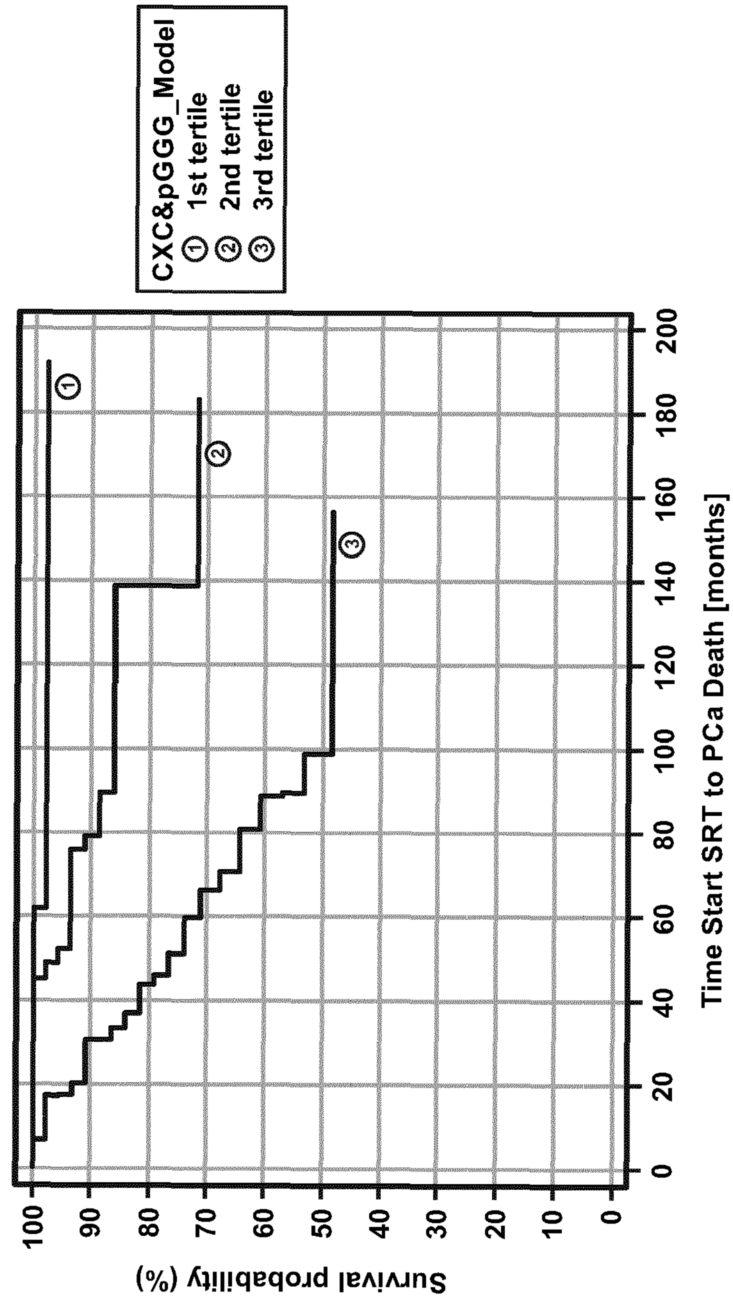
CXC&pGGG_Model
① 1st tertile
② 2nd tertile
③ 3rd tertile
Survival probability (%)
100
90
80
70
60
50
40
30
20
10
0
0
20
40
60
80
100
120
140
160
180
200
Time Start SRT to PCa Death [months]
①
②
③

FIG. 6

Europäisches Patentamt
European Patent Office
Office européen des brevets

# EUROPEAN SEARCH REPORT

Application Number
EP 20 16 4814

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/189403 A1 (INST NAT SANTE RECH MED [FR]; UNIV NICE SOPHIA ANTIPOLIS [FR] ET AL.) 18 October 2018 (2018-10-18) * page 45 - page 46 * | 12,13,15 | INV. C12Q1/6886 |
| X | WO 2015/048801 A2 (UNIV CALIFORINIA [US]) 2 April 2015 (2015-04-02) * paragraph [0094] - paragraph [0095] * | 12,13,15 | |
| A | WO 2019/028285 A2 (GENOMEDX INC [US]; UNIV MICHIGAN REGENTS [US]) 7 February 2019 (2019-02-07) | 1-16 | |
| A | ZHANG J ET AL: "CC chemokine ligand 2 (CCL2) promotes prostate cancer tumorigenesis and metastasis", CYTOKINE AND GROWTH FACTOR REVIEWS, ELSEVIER LTD, GB, vol. 21, no. 1, 1 February 2010 (2010-02-01), pages 41-48, XP026938191, ISSN: 1359-6101, DOI: 10.1016/J.CYTOGFR.2009.11.009 [retrieved on 2010-02-01] | 1-16 | |
| A | KOUJI IZUMI ET AL: "Serum chemokine (CC motif) ligand 2 level as a diagnostic, predictive, and prognostic biomarker for prostate cancer", ONCOTARGET, vol. 7, no. 7, 16 February 2016 (2016-02-16), pages 8389-8398, XP55732626, United States ISSN: 1949-2553, DOI: 10.18632/oncotarget.6690 | 1-16 | |

TECHNICAL FIELDS SEARCHED (IPC)
C12Q

-/--

1

The present search report has been drawn up for all claims

| Place of search | Date of completion of the search | Examiner |
|---|---|---|
| The Hague | 21 September 2020 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 16 4814

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | HALL WILLIAM A ET AL: "Biomarkers of Outcome in Patients With Localized Prostate Cancer Treated With Radiotherapy", SEMINARS IN RADIATION ONCOLOGY, ELSEVIER, AMSTERDAM, NL, vol. 27, no. 1, 16 September 2016 (2016-09-16), pages 11-20, XP029847419, ISSN: 1053-4296, DOI: 10.1016/J.SEMRADONC.2016.09.001<br>----- | 1-9, 11-16 | |

TECHNICAL FIELDS SEARCHED (IPC)

The present search report has been drawn up for all claims

1

| Place of search | Date of completion of the search | Examiner |
|---|---|---|
| The Hague | 21 September 2020 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 16 4814

This annex lists the patent family members relating to the patent documents cited in the above-mentioned European search report. The members are as contained in the European Patent Office EDP file on
The European Patent Office is in no way liable for these particulars which are merely given for the purpose of information.

21-09-2020

| Patent document cited in search report | Publication date | Patent family member(s) | Publication date |
|---|---|---|---|
| WO 2018189403 A1 | 18-10-2018 | NONE | |
| WO 2015048801 A2 | 02-04-2015 | AU 2014324408 A1 | 07-04-2016 |
| | | CA 2925050 A1 | 02-04-2015 |
| | | CN 105593375 A | 18-05-2016 |
| | | EP 3052659 A2 | 10-08-2016 |
| | | JP 2016540033 A | 22-12-2016 |
| | | US 2016368995 A1 | 22-12-2016 |
| | | WO 2015048801 A2 | 02-04-2015 |
| WO 2019028285 A2 | 07-02-2019 | AU 2018310987 A1 | 27-02-2020 |
| | | CA 3072061 A1 | 07-02-2019 |
| | | EP 3662082 A2 | 10-06-2020 |
| | | WO 2019028285 A2 | 07-02-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRAY F. et al.** Global cancer statistics 2018: GLOBOCAN estimates of incidence and mortality worldwide for 36 cancers in 185 countries. *CA Cancer J Clin,* 2018, vol. 68 (6), 394-424
- Cancer Facts & Figures 2010. American Cancer Society, 2010
- CANCER FACTS & FIGURES 2010. ACS, 2010
- **GRIMM P. et al.** Comparative analysis of prostate-specific antigen free survival outcomes for patients with low, intermediate and high risk prostate cancer treatment by radical therapy. Results from the Prostate Cancer Results Study Group. *BJU Int,* 2012, 22-29
- **GRIMM P et al.** *BJU INT,* 2012
- BJU INT. ACS, 2010
- **GRIMM P. et al.** *BJU INT,* 2012
- **DAL PRA A. et al.** Contemporary role of postoperative radiotherapy for prostate cancer. *Transl Androl Urol,* 2018, vol. 7 (3), 399-413
- **HERRERA F.G. ; BERTHOLD D.R.** Radiation therapy after radical prostatectomy: Implications for clinicians. *Front Oncol,* 2016, vol. 6 (117
- **HERRERA F.G. ; BERTHOLD D.R.** *FRONT ONCOL,* 2016
- **PISANSKY T.M. et al.** Salvage radiation therapy dose response for biochemical failure of prostate cancer after prostatectomy - A multi-institutional observational study. *Int J Radiat Oncol Biol Phys,* 2016, vol. 96 (5), 1046-1053
- **RESNICK M.J. et al.** Long-term functional outcomes after treatment for localized prostate cancer. *N Engl J Med,* 2013, vol. 368 (5), 436-445
- **HEGARTY S.E. et al.** Radiation therapy after radical prostatectomy for prostate cancer: Evaluation of complications and influence of radiation timing on outcomes in a large, population-based cohort. *PLoS One,* 2015, vol. 10 (2
- **PARAVATI A.J. et al.** Variation in the cost of radiation therapy among medicare patients with cancer. *J Oncol Pract,* 2015, vol. 11 (5), 403-409
- **HALL W.A. et al.** Biomarkers of outcome in patients with localized prostate cancer treated with radiotherapy. *Semin Radiat Oncol,* 2016, vol. 27, 11-20
- **RAYMOND E. et al.** An appraisal of analytical tools used in predicting clinical outcomes following radiation therapy treatment of men with prostate cancer: A systematic review. *Radiat Oncol,* 2017, vol. 12 (1), 56
- **HERRERA F.G. ; BERTHOLD D.R.** *RADIAT ONCOL,* 2016
- **HALL W.A. et al.** *RADIAT ONCOL,* 2016
- **DAL PRA A. et al.** *RADIAT ONCOL,* 2018
- **MANTOVANI A. et al.** Cancer-related inflammation. *Nature,* 2008, vol. 454 (7203), 436-444
- **GIRALDO N.A. et al.** The clinical role of the TME in solid cancer. *Br J Cancer,* 2019, vol. 120 (1), 45-53
- **GIRALDO N.A. et al.** *BR J CANCER,* 2019
- **HALL W.A. et al.** *BR J CANCER,* 2016
- **SHIAO S.L. et al.** Regulation of prostate cancer progression by tumor microenvironment. *Cancer Lett,* 2016, vol. 380 (1), 340-348
- **BARKER H.E. et al.** The tumor microenvironment after radiotherapy: Mechanisms of resistance or recurrence. *Nat Rev Cancer,* 2015, vol. 15 (7), 409-425
- **NAGARSHETH N. et al.** Chemokines in the cancer microenvironment and their relevance in cancer immunotherapy. *Nat Rev Immunol,* 2017, vol. 17 (9), 559-572
- **NAGARSHETH N. et al.** *NAT REV IMMUNOL,* 2017
- **HERAS S.C. LAS ; MARTINEZ-BALIBREA E.** CXC family of chemokines as prognostic or predictive biomarkers and possible drug targets in colorectal cancer. *World Journal of Gastroenterology,* 2018, vol. 24 (42), 4738-4749
- **HERRERA F.G. et al.** Radiotherapy combination opportunities leveraging immunity for the next oncology practice. *CA: A Cancer Journal for Clinicians,* 2017, vol. 67 (1), 65-85
- **JAROSZ-BIEJ M. et al.** Tumor microenvironment as a "game changer" in cancer radiotherapy. *IJMS,* 2019, vol. 20 (13), 3212
- **CONNOLLY K.A. et al.** Increasing the efficacy of radiotherapy by modulating the CCR2/CCR5 chemokine axes. *Oncotarget,* 2016, vol. 7 (52), 86522-86535
- **TSAUR I. et al.** CCL2 chemokine as a potential biomarker for prostate cancer: A pilot study. *Cancer Res Treat,* 2014, vol. 47 (2), 306-312
- **RENTOFT M. et al.** Expression of CXCL10 is associated with response to radiotherapy and overall survival in squamous cell carcinoma of the tongue. *Tumor Biol,* 2014, vol. 35 (5), 4191-4198

- **COOPERBERG M.R. et al.** The CAPRA-S score: A straightforward tool for improved prediction of outcomes after radical prostatectomy. *Cancer,* 2011, vol. 117 (22), 5039-5046

SEQUENCE LISTING

<110> Koninklijke Philips N.V.

<120> Prediction of radiotherapy response for prostate cancer subject based on chemokine genes

<130> 2020PF00063

<160> 4

<170> PatentIn version 3.5

<210> 1
<211> 741
<212> DNA
<213> Homo sapiens

<400> 1
gagaggctga gactaaccca gaaacatcca attctcaaac tgaagctcgc actctcgcct 60

ccagcatgaa agtctctgcc gcccttctgt gcctgctgct catagcagcc accttcattc 120

cccaagggct cgctcagcca gatgcaatca atgccccagt cacctgctgt tataacttca 180

ccaataggaa gatctcagtg cagaggctcg cgagctatag aagaatcacc agcagcaagt 240

gtcccaaaga agctgtgatc ttcaagacca ttgtggccaa ggagatctgt gctgacccca 300

agcagaagtg ggttcaggat tccatggacc acctggacaa gcaaacccaa actccgaaga 360

cttgaacact cactccacaa cccaagaatc tgcagctaac ttattttccc ctagctttcc 420

ccagacaccc tgttttattt tattataatg aattttgttt gttgatgtga aacattatgc 480

cttaagtaat gttaattctt atttaagtta ttgatgtttt aagtttatct ttcatggtac 540

tagtgttttt tagatacaga gacttgggga aattgctttt cctcttgaac cacagttcta 600

cccctgggat gttttgaggg tctttgcaag aatcattaat acaaagaatt tttttaaca 660

ttccaatgca ttgctaaaat attattgtgg aaatgaatat tttgtaacta ttacaccaaa 720

taaatatatt tttgtacaaa a 741

<210> 2
<211> 99
<212> PRT
<213> Homo sapiens

<400> 2

Met Lys Val Ser Ala Ala Leu Leu Cys Leu Leu Leu Ile Ala Ala Thr
1 5 10 15

Phe Ile Pro Gln Gly Leu Ala Gln Pro Asp Ala Ile Asn Ala Pro Val
20 25 30

Thr Cys Cys Tyr Asn Phe Thr Asn Arg Lys Ile Ser Val Gln Arg Leu
35 40 45

```
Ala Ser Tyr Arg Arg Ile Thr Ser Ser Lys Cys Pro Lys Glu Ala Val
    50                  55                  60

Ile Phe Lys Thr Ile Val Ala Lys Glu Ile Cys Ala Asp Pro Lys Gln
65                  70                  75                  80

Lys Trp Val Gln Asp Ser Met Asp His Leu Asp Lys Gln Thr Gln Thr
                85                  90                  95

Pro Lys Thr
```

<210> 3
<211> 938
<212> DNA
<213> Homo sapiens

<400> 3

```
gattcccata aagcacatgg tctaatctgt tacgtaacag caagacagcg tcacctcacc      60

tgttctcgcc ctcaaatggg aacgctggcc tgggactaaa gcatagacca ccaggctgag     120

tatcctgacc tgagtcatcc ccagggatca ggagcctcca gcagggaacc ttccattata     180

ttcttcaagc aacttacagc tgcaccgaca gttgcgatga aagttctaat ctcttccctc     240

ctcctgttgc tgccactaat gctgatgtcc atggtctcta gcagcctgaa tccaggggtc     300

gccagaggcc acagggaccg aggccaggct tctaggagat ggctccagga aggcggccaa     360

gaatgtgagt gcaaagattg gttcctgaga gccccgagaa gaaaattcat gacagtgtct     420

gggctgccaa agaagcagtg cccctgtgat catttcaagg gcaatgtgaa gaaaacaaga     480

caccaaaggc accacagaaa gccaaacaag cattccagag cctgccagca atttctcaaa     540

caatgtcagc taagaagctt tgctctgcct ttgtaggagc tctgagcgcc cactcttcca     600

attaaacatt ctcagccaag aagacagtga gcacacctac cagacactct tcttctccca     660

cctcactctc ccactgtacc cacccctaaa tcattccagt gctctcaaaa agcatgtttt     720

tcaagatcat tttgtttgtt gctctctcta gtgtcttctt ctctcgtcag tcttagcctg     780

tgccctcccc ttacccaggc ttaggcttaa ttacctgaaa gattccagga aactgtagct     840

tcctagctag tgtcatttaa ccttaaatgc aatcaggaaa gtagcaaaca gaagtcaata     900

aatattttta aatgtcacag ataaaaaaaa aaaaaaaa                            938
```

<210> 4
<211> 119
<212> PRT
<213> Homo sapiens

<400> 4

```
Met Lys Val Leu Ile Ser Ser Leu Leu Leu Leu Leu Pro Leu Met Leu
1               5                   10                  15

Met Ser Met Val Ser Ser Ser Leu Asn Pro Gly Val Ala Arg Gly His
            20                  25                  30

Arg Asp Arg Gly Gln Ala Ser Arg Arg Trp Leu Gln Glu Gly Gly Gln
        35                  40                  45

Glu Cys Glu Cys Lys Asp Trp Phe Leu Arg Ala Pro Arg Arg Lys Phe
    50                  55                  60

Met Thr Val Ser Gly Leu Pro Lys Lys Gln Cys Pro Cys Asp His Phe
65                  70                  75                  80

Lys Gly Asn Val Lys Lys Thr Arg His Gln Arg His His Arg Lys Pro
                85                  90                  95

Asn Lys His Ser Arg Ala Cys Gln Gln Phe Leu Lys Gln Cys Gln Leu
            100                 105                 110

Arg Ser Phe Ala Leu Pro Leu
        115
```